# EUROPEAN PATENT APPLICATION

(11) **EP 4 279 120 A1**
(43) Date of publication of application: **22.11.2023**
(21) Application number: 22173559.0
(22) Date of filing: 16.05.2022
(51) Int. Cl.: A61N 1/36, A61N 1/05

(54) **A DEVICE FOR STIMULATING A NERVE AND A METHOD FOR CONTROLLING STIMULATION**

(71) Applicant: Stichting IMEC Nederland, 5656 AE Eindhoven (NL)
(72) Inventor: FICHMAN, Mark, 5654 KC Eindhoven (NL); MIHAJLOVIC, Vojkan, 5643 AZ Eindhoven (NL)
(74) Representative: AWA Sweden AB

(57) **Abstract**

A device (100; 200; 300) for stimulating a nerve (10) comprising: a first stimulation generating unit (110; 210; 310) configured to generate and output a first intermittent current waveform (116) comprising a sequence of first pulses (116a, 116b) to a first pair of electrodes (114; 214; 314); a second stimulation generating unit (130; 230; 330) configured to generate and output a second intermittent current waveform (136) comprising a sequence of second pulses (136a, 136b) to a second pair of electrodes (134; 234; 334); wherein the first (116) and second intermittent current waveform (136) have a difference in frequency so as to stimulate the nerve (10) using interferential stimulation based on interference between the first (116) and second intermittent current waveform (136); and a control unit (160; 260; 360) configured to control the interferential stimulation and synchronize the first (110; 210; 310) and the second stimulation generating unit (130; 230; 330).

## Description

### Technical field

The present description relates to stimulation of a nerve of a living being. In particular, the present description relates to a device for stimulating a nerve and to a method for controlling stimulation.

### Background

Neurostimulation may be used for controlling a function of an organ or part of a body of a living being, such as a human being, e.g., for alleviating or treating a number of conditions of the human being. Neurostimulation may involve a control of electrical signals transported through a nerve, so as to trigger or block signals transported by the nerve.

Stimulation of a nerve may be provided using a plurality of electrodes arranged in different locations in relation to the nerve. In particular, if it is desired to target a specific part of the nerve, such as a fascicle or nerve fiber(s) within the fascicle, use of the plurality of electrodes is advantageous. Typically, a signal provided to a nerve will attenuate with a depth into the nerve such that a maximum electric field is provided at an outer part of the nerve, in epineurium. In particular, since the epineurium has low conductive properties, large signals are required for reaching deep into the nerve. However, signals from two pairs of electrodes may be provided so as to interfere within the nerve and selectively provide a large electric field within the nerve, forming interferential stimulation of the nerve. This implies that a part of the nerve inside the epineurium may be stimulated while using modest signal levels.

However, if stimulation of the nerve is to be provided in a stimulation period extending for a long period of time, the stimulation requires a large amount of power, in particular for driving two pairs of electrodes for forming interferential stimulation. Thus, there is a need for an energy efficient stimulation of the nerve. This is particularly important if a device is to be implanted within the body of the living being.

### Summary

An objective of the description is to provide an energy efficient stimulation of a nerve. Another objective of the description is to provide stimulation of the nerve which may be accurately controlled.

These and other objectives are at least partly met by the invention as defined in the independent claims. Preferred embodiments are set out in the dependent claims.

According to a first aspect, there is provided a device for stimulating a nerve of a living being, said device comprising: a first stimulation generating unit comprising a first current generator and a first pair of electrodes configured to be arranged in a first location in relation to the nerve, wherein the first current generator is configured to generate and output a first intermittent current waveform comprising a sequence of first pulses to the first pair of electrodes; a second stimulation generating unit comprising a second current generator and a second pair of electrodes configured to be arranged in a second location in relation to the nerve, wherein the second current generator is configured to generate and output a second intermittent current waveform comprising a sequence of second pulses to the second pair of electrodes; wherein the first stimulation generating unit and the second stimulation generating unit are configured to generate and output the first intermittent current waveform and the second intermittent current waveform with a difference in frequency of a first pulse in the sequence of first pulses and a second pulse in the sequence of second pulses such that the device is configured to stimulate the nerve using interferential stimulation based on interference between the first intermittent current waveform and the second intermittent current waveform; and a control unit configured to control the first intermittent current waveform and the second intermittent current waveform for controlling the interferential stimulation and configured to synchronize the first stimulation generating unit and the second stimulation generating unit for controlling output of the first pulses and the second pulses.

The device comprises a first stimulation generating unit and a second stimulation generating units with separate current generators. This implies that the first waveform to be output to the first pair of electrodes and the second waveform to be output to the second pair of electrodes may be separately controlled. Hence, the interferential stimulation may be accurately controlled by separately controlling the first waveform and the second waveform.

The device is configured to provide a first intermittent current waveform and a second intermittent current waveform. Thus, a signal may be intermittently provided to the first pair of electrodes and a signal may be intermittently provided to the second pair of electrodes. The first waveform and the second waveform need not be continuous over a plurality of pulses with the first waveform and/or the second waveform being modulated to form pulses of interferential stimulation with sufficient amplitude to activate the nerve. Rather, the first pair of electrodes and the second pair of electrodes may only receive signals at time instances when stimulation of the nerve is to take place.

Thus, the first intermittent current waveform may provide a non-zero signal or high amplitude signal within the first pulses and may provide a zero-level signal between the first pulses within the sequence of first pulses. Also, the second intermittent current waveform may provide a non-zero signal or high amplitude signal within the second pulses and may provide a zero-level signal between the second pulses within the sequence of second pulses. It should be realized that instead of a zero-level signal a low amplitude signal may be provided between the first pulses and between the second pulses, respectively. The low amplitude signal may have such a low amplitude that no stimulation of the nerve takes place through interferential stimulation and the amplitude may also be low so as not to draw a lot of energy.

The first intermittent current waveform and the second intermittent current waveform may thus be called intermittent in that the waveforms intermittently have sufficiently high amplitude to cause interferential stimulation and therebetween have a very low or zero amplitude to reduce power consumption.

Thus, thanks to the stimulation generating units being configured to provide intermittent current waveforms, the stimulation of the nerve may be provided in an energy-efficient manner. The stimulation generating units need only output signals during the first pulses and the second pulses, respectively.

This implies that the device facilitates use of an implanted device (for which energy saving is particularly important in order to ensure long lifetime of the implanted device or avoid frequent charging of the implanted device) with stimulation paradigms that include a nerve being stimulated in very long sessions (such as up to 30 minutes) comprising sequences of pulses being provided in plural periods separated by "off time" when no stimulation is provided.

Thanks to the control unit providing synchronization of the first stimulation generating unit and the second stimulation generating unit, the first stimulation generating unit and the second stimulation generating unit are controlled so as to provide the first pulses and the second pulses with a desired relationship in time for ensuring desired interferential stimulation of the nerve based on the interference between a first pulse and a second pulse.

The first intermittent current waveform comprises a varying signal defining a first frequency within each of the first pulses. Thus, the first frequency is provided within the first pulse. This should not be confused with a periodicity of the pulses, defining a time interval between two different pulses within the sequence of first pulses.

The interferential stimulation is based on the difference in frequency of a first pulse in the sequence of first pulses and a second pulse in the sequence of second pulses. When the first intermittent current waveform and the second intermittent current waveform interfere, the interference may form a varying signal having a frequency corresponding to the difference in frequency of a first pulse in the sequence of first pulses and a second pulse in the sequence of second pulses. This frequency of the interferential signal may be referred to as a beat frequency.

The first frequency and the second frequency may be selected to be high frequencies, whereas the beat frequency represents a low frequency lower than the first frequency and the second frequency. The nerve may be affected to a larger extent by low frequency signals. Thus, the first intermittent current waveform and the second intermittent current waveform may include a first frequency and a second frequency, respectively, which do not affect, or minimally affect, the nerve. Further, an amplitude of the varying signal defining the first frequency and an amplitude of the varying signal defining the second frequency may be smaller than an amplitude of an interferential signal with the beat frequency, such that a sufficiently large signal (amplitude) for stimulating the nerve is only provided by the interferential signal. Hence, the stimulation of the nerve may be solely provided by the interferential signal.

Similarly, the second intermittent current waveform comprises a varying signal defining a second frequency within each of the second pulses. Thus, the second frequency is provided within the second pulse. This should not be confused with a periodicity of the pulses, defining a time interval between two different pulses within the sequence of second pulses.

The device may be used for stimulating a nerve of a living being. It should be realized that the device may be used for stimulating a nerve of a human being but that the device may likewise be used for stimulating nerves of animals.

As used herein, the term "nerve" should be construed as a bundle of nerve fibers enclosed by a sheath called the epineurium. The nerve may form part of the peripheral nervous system and may therefore also be called a peripheral nerve. The nerve is configured to transmit electrical impulses within the body along axons, which may be bundled in fascicles in fascicles in the nerve. The electrical impulses may be provided as action potentials transmitted along the axons.

The nerve may comprise a plurality of fascicles. Within the nerve, a large number of fascicles may be provided, and each fascicle may include a large number of axons, such that the nerve may transmit signals to / from various parts of the body. For instance, the vagus nerve is a cranial nerve providing signals between the brain and parts of the body. The vagus nerve may be relatively easy to access and a device for stimulating a nerve that is to be implanted in the human being may therefore be advantageously arranged in relation to the vagus nerve for providing stimulation of the vagus nerve. However, since the vagus nerve provides signals to many different parts of the body, it is also important that stimulation of the nerve is controlled through interferential stimulation to selectively stimulate a portion of the nerve for providing a desired effect of the stimulation.

Stimulation of the nerve may correspond to providing a sufficiently large action potential so as to trigger a signal to be transported by the nerve or block a signal from being transported by the nerve.

A stimulation paradigm may define a desired beat frequency, a number of pulses in the sequence of pulses, duration of pulses, duration between pulses within a sequence of pulses, duration of "off time" between sequences of pulses, number of sequences of pulses, etc. The stimulation paradigm may be defined for providing a desired effect of the stimulation of the nerve, such as for providing control of a part of the body or an organ, or for providing treatment of a condition of the living being, such as treatment of epilepsy or depression of a human being.

Reference is made herein to "first" and "second", such as "first stimulation generating unit" and "second stimulation generating unit". It should be realized that items referred to as "first" are not the same item as items referred to as "second". Thus, the first stimulation generating unit is different from the second stimulation generating unit. The first current generator is different from the second current generator. The first pair of electrodes is different from the second pair of electrodes. The first location in relation to the nerve is different from the second location in relation to the nerve. The first intermittent current waveform is different from the second intermittent current waveform. The first pulses are different from the second pulses.

The stimulation generating units are configured to generate current waveforms for providing a current to the nerve. The current can be converted to a voltage within the nerve based on transconductance of tissue such that an action potential for stimulating the nerve is formed.

According to an embodiment, the device is configured to be implanted in the living being with the first pair of electrodes and the second pair of electrodes arranged at different locations in relation to the nerve.

Thus, the device is adapted to provide the pairs of electrodes close to or in contact with the nerve to be stimulated. This implies that stimulation signals may be output by the device directly into the nerve providing an accurate control of the stimulation within the nerve.

The device may be configured to be completely implanted within the body of the living being. This facilitates use of the implanted device for providing stimulation of the nerve to the living being in a convenient way. In particular, when the device is implanted within the body of a human being, the stimulation of the nerve may be provided with minimal effect of everyday life of the human being for providing the stimulation.

However, the implanted device may still be configured to communicate with an external device through wireless communication. The device may receive control signals from the external device for changing parameters of the stimulation, such as parameters defining the stimulation paradigm, such as to control a location within the nerve to be stimulated or a frequency, an amplitude, or a duration of a stimulation to be provided. The external device may be provided in a housing that may be worn by the living being or may be adapted for being arranged in vicinity of the living being. The implanted device may be configured to communicate with a communication unit, which may be provided in a housing worn by the living being or arranged in vicinity of the living being, wherein the communication unit may act as an intermediate device for allowing the implanted device to communicate with an external unit which may be remotely arranged. Thus, communication between the communication unit and the external unit may be provided via a telecommunication or computer network.

It should further be realized that according to another embodiment, the control unit may be arranged in a separate housing from the first stimulation generating unit and the second stimulation generating unit. The control unit may then for instance be arranged in a housing that may be configured to be worn by the living being. The control unit may then be configured to provide control of the first stimulation generating unit and the second stimulation generating unit through wireless communication.

According to an embodiment, the electrodes of the first pair of electrodes and the second pair of electrodes are displaced in relation to each other along a longitudinal direction of the nerve. For instance, the electrodes of the first pair of electrodes may be arranged in different longitudinal locations in relation to the nerve. Also, the electrodes of the second pair of electrodes may be arranged in different longitudinal locations in relation to the nerve, which may be the same longitudinal positions as the longitudinal positions of the electrodes of the first pair of electrodes. The first intermittent current waveform and the second intermittent current waveform may thus each define electric fields along a longitudinal direction of the nerve extending into the nerve so as to form interferential stimulation.

According to another embodiment, the first pair of electrodes and the second pair of electrodes are arranged at different locations of a circumference of the nerve. Thus, the first intermittent current waveform and the second intermittent current waveform may mainly define electric fields based on the waveforms in different portions of a cross-section of the nerve. The first intermittent current waveform and the second intermittent current waveform may interfere within the nerve to stimulate the nerve through interferential stimulation.

Both electrodes of the first pair of electrodes may be associated with a first part of the circumference of the nerve and both electrodes of the second pair of electrodes may be associated with a second part of the circumference of the nerve, wherein the second part is different from and non-overlapping with the first part. This implies that the portions of the cross-section of the nerve in which the first intermittent current waveform and the second intermittent current waveform, respectively, define electric fields are quite different so as to allow interferential stimulation deep within the nerve without necessarily providing stimulation in undesired locations of the nerve.

Alternatively, the electrodes of the first pair of electrodes and the electrodes of the second pair of electrodes may be alternatingly arranged around the circumference of the nerve, such that an electrode of the first pair of electrodes is arranged between the electrodes of the second pair of electrodes along the circumference of the nerve. This implies that the first intermittent current waveform and the second intermittent current waveform may define electric fields through the nerve with a large area of overlap of the electric fields based on the first intermittent current waveform and the second intermittent current waveform, respectively.

All of the electrodes of the first pair of electrodes and the second pair of electrodes may be provided in relation to a common cross-section of the nerve.

According to an embodiment, the device comprises a cuff configured to be arranged surrounding the circumference of the nerve, wherein the first pair of electrodes and the second pair of electrodes are mounted in or on the cuff.

The cuff provides a convenient manner of arranging the electrodes in relation to a cross-section of the nerve. The cuff may be easily mounted around the nerve, whereby the cuff will position the electrodes of the first pair of electrodes and the second pair of electrodes in appropriate positions in relation to the nerve. This facilitates implantation of the device.

According to an embodiment, the control unit is configured to control the first stimulation generating unit and the second stimulation generating unit for turning on and off the first stimulation generating unit and the second stimulation generating unit, wherein the control unit is configured to turn off the first stimulation generating unit and the second stimulation generating unit between generation of individual pulses in the sequence of first pulses and in the sequence of second pulses.

The first stimulation generating unit and the second stimulation generating unit only provide output when the interference between the first intermittent current waveform and the second intermittent current waveform actually should affect the nerve. Hence, the device provides stimulation of the nerve in an energy-efficient manner.

The first stimulation generating unit and the second stimulation generating unit may be turned off between generation of individual pulses, so that the first stimulation generating unit and the second stimulation generating unit do not consume any power at all between generation of individual pulses.

Alternatively, the first stimulation generating unit and the second stimulation generating unit may receive power but may be deactivated and provide no output between individual pulses so as to save power. Thus, the control unit may be configured to activate the first stimulation generating unit and the second stimulation generating unit when individual pulses are to be output and may be configured to deactivate the first stimulation generating unit and the second stimulation generating unit between individual pulses in the sequency of first pulses and the sequence of second pulses. The first stimulation generating unit and the second stimulation generating unit may be completely turned off between different periods for outputting sequences of pulses, such that the first stimulation generating unit and the second stimulation generating unit may be turned off during the "off time" between periods of stimulation. The "off time" is typically longer than a time duration between individual pulses in a sequence of pulses, such that power is saved by completely turning off the first stimulation generating unit and the second stimulation generating unit during the "off time".

According to an embodiment, the control unit is configured to modulate the first intermittent current waveform and the second intermittent current waveform by modulating a frequency of the first pulses and a frequency of the second pulses, respectively, by modulating an amplitude of the first pulses and an amplitude of the second pulses, respectively, and/or by modulating a time instant for start of the first pulses and a time instant for start of the second pulses, respectively.

Thus, the control unit being configured to control the first intermittent current waveform and the second intermittent current waveform comprises the control unit being configured to modulate the first intermittent current waveform and the second intermittent current waveform.

The first current generator and the second current generator may be configured to generate an alternating signal. The control unit may be configured to provide a control signal to the first current generator and the second current generator, respectively, for modulating the alternating signal output by the first current generator and the second current generator, respectively.

The control unit may be configured to modulate a first frequency of the alternating signal within the first pulses and/or to modulate a second frequency of the alternating signal within the second pulses. This implies that the beat frequency may be controlled, which may be used for controlling stimulation of the nerve.

The control unit may also or alternatively be configured to modulate an amplitude of the first pulses and an amplitude of the second pulses. This implies that a location in the nerve having a maximum amplitude of the interferential signal may be controlled. If the amplitude of the first pulses is increased, a location having maximum amplitude of the interferential signal is moved closer to the second pair of electrodes, and vice versa. Thus, by modulating the amplitude of the first pulses and the amplitude of the second pulses, a location within the nerve where stimulation is provided may be controlled. This implies that the control unit may be configured to selectively stimulate a particular fascicle or even a particular axon or group of axons within the nerve.

The control unit may also or alternatively be configured to modulate a time instant for start of the first pulses and a time instant for start of the second pulses, respectively. Thus, the control unit may be configured to control a phase relation between the signals in the first pulses and the second pulses, which further controls a waveform of the interferential signal.

According to an embodiment, the first stimulation generating unit and the second stimulation generating unit are configured to generate a frequency of the first pulse and a frequency of the second pulse, respectively, being in a range of 500 Hz - 1 MHz, such as 50 kHz - 1 MHz.

Thus, the first stimulation generating unit and the second stimulation generating unit may generate signals having a relatively high frequency. The frequency of the signal in the first pulse and the frequency of the signal in the second pulse may be so high as not to affect, or minimally affect, the nerve. By selecting these frequencies to be high, such as in the range of 5 kHz - 1 MHz or in the range of 50 kHz - 1 MHz, the nerve is not stimulated by the first pulses and the second pulses individually. Rather, stimulation of the nerve may be provided solely by the interferential signal between the first intermittent current waveform and the second intermittent current waveform.

The frequency of the signal in the first pulse and the frequency of the signal in the second pulse may preferably be sufficiently high so as not to affect the nerve, such as being above 500 Hz, or preferably above 50 kHz. Also, the amplitude of the signal in the first pulse and the signal in the second pulse may be relatively low compared to the amplitude of the interferential signal, such that the relatively low amplitude signal in the first and second pulses, respectively, does not affect the nerve. The frequency of the signal in the first pulse and the frequency of the signal in the second pulse may preferably not be too high so that a relatively small difference in the frequencies may still be accurately defined. Thus, the frequency of the signal in the first pulse and the frequency of the signal in the second pulse may be lower than 1 MHz, such as lower than 100 kHz.

According to an embodiment, the first stimulation generating unit and the second stimulation generating unit are configured to generate the first intermittent current waveform and the second intermittent current waveform with a difference in frequency in a range of 1 Hz - 10 kHz, such as 1 - 5 kHz.

Thus, the beat frequency may be in the range of 1 Hz - 10 kHz or in the range of 1 - 5 kHz. The use of a beat frequency in these ranges may be suitable for providing stimulation of the nerve. If the beat frequency is very low, it may be difficult to accurately define the interferential signal in relation to high frequencies of the signal in the first pulse and the signal in the second pulse. Thus, the beat frequency may preferably be higher than 1 Hz or higher than 1 kHz. If the beat frequency is too high, the interferential signal may not provide a strong effect for stimulation of the nerve. Thus, the beat frequency may preferably be lower than 10 kHz or lower than 5 kHz.

The beat frequency may be selected in relation to a desired stimulation of the nerve, such as in relation to a particular stimulation paradigm to be used.

According to an embodiment, each of the first current generator and the second current generator comprises an oscillator, which is configured to generate an alternating signal and an amplifier, which is configured to receive the alternating signal from the oscillator and generate a source current to a first electrode and a sink current to a second electrode in the first pair of electrodes and the second pair of electrodes, respectively.

Thus, the first current generator may comprise a first oscillator and a first amplifier and the second current generator may comprise a second oscillator and a second amplifier.

The oscillator may be configured to generate the alternating signal with an accurately defined frequency. The amplifier may be configured to provide a source current and a sink current to drive the pair of electrodes.

According to an embodiment, the control unit is configured to transfer control signals to the first stimulation generating unit and the second stimulation generating unit via inductive couplings.

This implies that the transfer of control signals to the first stimulation generating unit and the second stimulation generating unit, respectively, is independently provided.

The inductive couplings between the control unit and the first stimulation generating unit and between the control unit and the second stimulation generating unit imply that the first stimulation generating unit and the second stimulation generating unit are galvanically isolated. This implies that crosstalk between the first stimulation generating unit and the second stimulation generating unit is avoided such that the interferential stimulation is provided by interference between the first intermittent current waveform and the second intermittent current waveform and is not affected by any crosstalk between the first stimulation generating unit and the second stimulation generating unit.

Each inductive coupling may be provided by a pair of coils arranged close to each other. Thus, the control unit may provide a control signal through a first coil in the pair of coils and the first stimulation generating unit and the second stimulation generating unit, respectively, may receive the control signal in a second coil in the pair of coils.

The source current generated by the first current generator and the source current generated by the second current generator may have a floating potential in relation to each other. This further avoids crosstalk between the first stimulation generating unit and the second stimulation generating unit.

According to an embodiment, the first stimulation generating unit is configured to provide a source current signal to a first electrode in the first pair of electrodes and to provide a sink current signal to a second electrode in the first pair of electrodes, wherein the control unit is configured to transmit a first control signal to the first stimulation generating unit for setting frequency of the first stimulation generating unit and to transmit a second control signal to the first stimulation generating unit for controlling start of the source current signal and the sink current signal.

This implies that the first electrode may actively source a current into the nerve and the second electrode may actively sink a same amount of current. The first stimulation generating unit may be controlled by two control signals for setting the frequency and controlling the source current signal and the sink current signal. This may be used for avoiding crosstalk between the first stimulation generating unit and the second stimulation generating unit even if the first stimulation generating unit and the second stimulation generating unit are not galvanically isolated.

Similarly, the second stimulation generating unit may be configured to provide a source current signal to a first electrode in the second pair of electrodes and to provide a sink current signal to a second electrode in the second pair of electrodes. The control unit may further be configured to transmit a third control signal to the second stimulation generating unit for setting frequency of the second stimulation generating unit and to transmit a fourth control signal to the second stimulation generating unit for controlling start of the source current signal and the sink current signal provided by the second stimulation generating unit.

According to an embodiment, the first pulses and second pulses are sinusoidal signals.

This implies that first pulses and the second pulses do not deliver net charges to the living being. Hence, accumulation of net charges in the living being is avoided, which may otherwise negatively affect the living being.

According to an embodiment, the device further comprises a third stimulation generating unit comprising a third current generator and a third pair of electrodes configured to be arranged in a third location in relation to the nerve, wherein the third current generator is configured to generate and output a third intermittent current waveform comprising a sequence of third pulses to the third pair of electrodes, wherein the control unit is further configured to control the third intermittent current waveform for controlling the interferential stimulation based on the first, second, and third intermittent current waveforms.

Thus, in addition to the first stimulation generating unit and the second stimulation generating unit, the device may further comprise one or more additional stimulation generating units, such as a third stimulation generating unit.

The third stimulation generating unit (and any further stimulation generating unit) may be individually controlled to provide a third intermittent current waveform independently of the first intermittent current waveform and the second intermittent current waveform.

The third intermittent current waveform may contribute to forming of the interferential stimulation so as to allow a further improved control of the interferential stimulation. The use of additional intermittent current waveforms, such as the third intermittent current waveform, may thus allow an improved control of the interferential stimulation.

The frequency, amplitude, and/or time instant for start of the third pulses may be controlled independently from control of the first intermittent current waveform and the second intermittent current waveform. The interferential stimulation may thus be controlled to obtain an arbitrary waveform of the interferential stimulation and to provide accurate location within the nerve wherein stimulation of the nerve is provided by the interferential stimulation.

The third stimulation generating unit may be configured to provide a different frequency of the signal of the third pulses from the frequencies of the first pulses and the second pulses, respectively. According to an embodiment, the frequency of the signal of the third pulses may be in a range of 500 Hz - 1 MHz, such as 50 kHz - 1 MHz. Alternatively, the frequency of the third pulses may be the same as the frequency of the first pulses or the frequency of the second pulses.

The third pair of electrodes may be arranged at a different location of a circumference of the nerve compared to the first pair of electrodes and the second pair of electrodes. All of the electrodes of the first pair of electrodes, the second pair of electrodes, and the third pair of electrodes may be arranged in relation to a common cross-section of the nerve. According to an alternative, the electrodes may also be displaced in relation to each other along a longitudinal direction of the nerve.

The third stimulation generating unit may be used such that the device may control interferential stimulation based on the first intermittent current waveform, the second intermittent current waveform, and the third intermittent current waveform being simultaneously output. However, the device may additionally or alternatively control interferential stimulation by the control unit selectively activating stimulation generating units so as to select which two of the first intermittent current waveform, the second intermittent current waveform, and the third intermittent current waveform are to be used for defining interferential stimulation. In other words, the control unit may be configured to select which electrodes that are to be used for outputting signals for defining the interferential stimulation.

Hence, the control unit may be configured to modulate interferential stimulation by selecting which electrodes to be used for output of waveforms for stimulation of the nerve. The selection of which electrodes to be used may be used for controlling a location within the nerve to be stimulated.

According to an embodiment, the device comprises a sensor for sensing a voltage amplitude at the first pair of electrodes and at the second pair of electrodes, respectively, wherein the control unit is configured to control the first stimulation generation unit and the second stimulation generation unit for controlling the voltage amplitude and controlling the interferential stimulation.

The first intermittent current waveform and the second intermittent current waveform generate a corresponding voltage across the first pair of electrodes and across the second pair of electrodes, respectively. This voltage may be sensed using the same pair of electrodes for providing the intermittent current waveforms into the nerve or using another pair of electrodes arranged in close vicinity to the first pair of electrodes and the second pair of electrodes, respectively.

By monitoring the voltage amplitude at the first pair of electrodes and the second pair of electrodes, respectively, feedback on the stimulation may be provided. The control unit may be configured to control the first stimulation generating unit and the second stimulation generating unit for controlling the amplitude of the first intermittent current waveform and the second intermittent current waveform, respectively, which in turn controls the corresponding voltage amplitude.

The interferential stimulation may be controlled so as to control a location within the nerve where stimulation is provided. This implies that the sensor may provide feedback for allowing the control unit to accurately control the location within the nerve where stimulation is provided.

According to a second aspect, there is provided a system for stimulating a nerve of a living being, said system comprising: a device according to the first aspect, wherein the device is configured to be implanted in the living being and the device comprises a communication unit for wireless communication; an external device configured to communicate through wireless communication with the communication unit of the device.

Effects and features of this second aspect are largely analogous to those described above in connection with the first aspect. Embodiments mentioned in relation to the second aspect are largely compatible with the first aspect.

Thanks to the system, an implanted device may be provided and may still be controlled after implantation via an external device. The external device may be used for communicating configuration settings to the implanted device so as to control the stimulation provided by the implanted device. Also, the implanted device may communicate information of stimulations performed and/or information of status of the implanted device to the external device.

The external device may function as an intermediate device providing communication between the implanted device and a remote device which may be arranged anywhere in relation to the living being. The external device and/or the remote device may further provide an interface for allowing user input for controlling the stimulation provided by the implanted device.

According to a third aspect, there is provided a method for controlling stimulation of a nerve of a living being, said method comprising: providing a first control signal for controlling a first stimulation generating unit comprising a first current generator and a first pair of electrodes arranged in a first location in relation to the nerve, wherein the control signal controls generation of a first intermittent current waveform comprising a sequence of first pulses; providing a second control signal for controlling a second stimulation generating unit comprising a second current generator and a second pair of electrodes arranged in a second location in relation to the nerve, wherein the control signal controls generation of a second intermittent current waveform comprising a sequence of second pulses; wherein the control signals to the first stimulation generating unit and the second stimulation generating unit controls generation of the first intermittent current waveform and the second intermittent current waveform with a difference in frequency of a first pulse in the sequence of first pulses and a second pulse in the sequence of second pulses; wherein the control signals are synchronized for synchronizing the first stimulation generating unit and the second stimulation generating unit.

Effects and features of this third aspect are largely analogous to those described above in connection with the first and second aspects. Embodiments mentioned in relation to the third aspect are largely compatible with the first and second aspects.

The method relates to controlling generation of signals that may be used for stimulation of the nerve. The method provides control signals for controlling timing of generation of the first intermittent current waveform and the second intermittent current waveform and for synchronizing the first stimulation generating unit and the second stimulation generating unit. Thus, the method provides control of a device for generating pulses. The method does not relate to actual output of pulses and is therefore not related to a method of treatment of a living being.

The providing of independent control signals to the first stimulation generating unit and the second stimulation generating unit implies that the stimulation generating units may be individually controlled. Thanks to using separate stimulation generating units, the first intermittent current waveform may be independently generated from the second intermittent current waveform. However, thanks to the control signals being synchronized, timing of the generation of the first intermittent current waveform in relation to generation of the second intermittent current waveform may still be controlled.

The method allows generation of intermittent current waveforms, such that current waveforms may provide a zero-level signal between pulses. Thus, thanks to the stimulation generating units being controlled to generate intermittent current waveforms, the stimulation of the nerve may be provided in an energy-efficient manner.

According to an embodiment, the method further comprises controlling the first stimulation generating unit and the second stimulation generating unit for turning on and off the first stimulation generating unit and the second stimulation generating unit, wherein the first stimulation generating unit and the second stimulation generating unit are turned off between generation of individual pulses in the sequence of first pulses and in the sequence of second pulses.

This implies that the control of the stimulation may ensure that stimulation is provided in a very energy-efficient manner as the first stimulation generating unit and the second stimulation generating unit do not consume any power at all between generation of individual pulses.

According to an embodiment, the method further comprises modulating the first intermittent current waveform and the second intermittent current waveform by modulating a frequency of the first pulses and a frequency of the second pulses, respectively, by modulating an amplitude of the first pulses and an amplitude of the second pulses, respectively, and/or by modulating a time instant for start of the first pulses and a time instant for start of the second pulses, respectively.

This implies that the control of stimulation may accurately control generation of the first intermittent current waveform and the second intermittent current waveform for accurately defining interferential stimulation.

### Brief description of the drawings

The above, as well as additional objects, features, and advantages of the present inventive concept, will be better understood through the following illustrative and non-limiting detailed description, with reference to the appended drawings. In the drawings like reference numerals will be used for like elements unless stated otherwise.
Fig. 1 is a schematic view illustrating interferential stimulation of a nerve.
Fig. 2 is a schematic view of a first intermittent current waveform and a second intermittent current waveform and an interferential signal formed by the first intermittent current waveform and the second intermittent current waveform according to an embodiment.
Fig. 3 is a schematic view of a device according to a first embodiment.
Fig. 4 is a schematic view of a device according to a second embodiment.
Fig. 5 is a schematic view of a device according to a third embodiment.
Fig. 6 is a flow chart of a method according to an embodiment.

### Detailed description

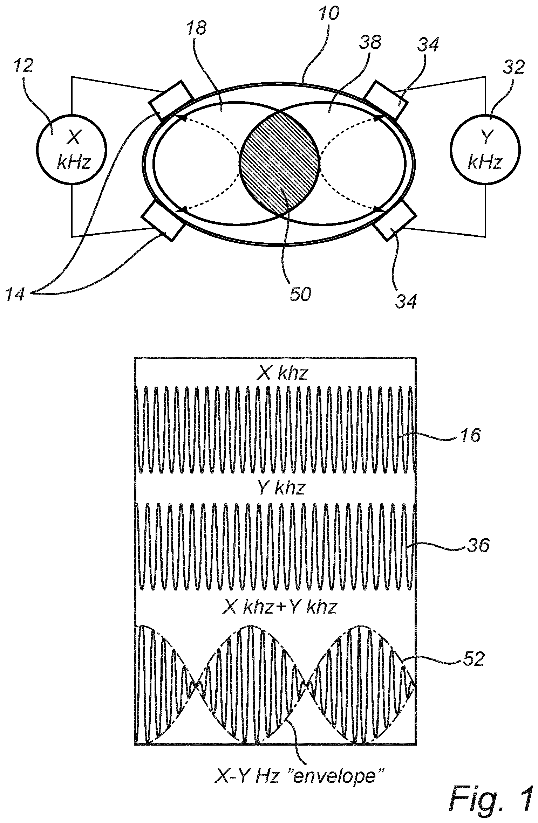

Referring now to Fig. 1, interferential stimulation of a nerve 10 will be generally described.

As illustrated in Fig. 1, a first current source 12 is associated with a first pair of electrodes 14 and a second current source 32 is associated with a second pair of electrodes 34. The first pair of electrodes 14 is mounted in a first relation to an outer surface of the nerve 10 and the second pair of electrodes 34 is mounted in a second relation to the outer surface of the nerve 10.

The first current source 12 generates a first signal 16 with a first frequency X kHz and the second current source 32 generates a second signal 36 with a second frequency Y kHz. As illustrated in Fig. 1, the first signal provided to the first pair of electrodes 14 causes an electric field to be generated in a first portion 18 of the nerve 10 and the second signal 36 provided to the second pair of electrodes 34 causes an electric field to be generated in a second portion 38 of the nerve 1. The electric fields overlap in an overlapping portion 50, wherein the first signal 16 interferes with the second signal 36 to generate an interferential signal 52.

The interferential signal 52 has a frequency content including the first frequency and the second frequency. Further, the amplitude of the interferential signal 52 is modulated by a difference between the first frequency and the second frequency, X-Y, defining a beat frequency of the interferential signal 52.

The beat frequency may be much lower than the first and second frequencies. Further, the amplitude of the interferential signal 52 may be larger than individual amplitudes of the first signal 16 and the second signal 36, respectively, thanks to constructive interference of the signals.

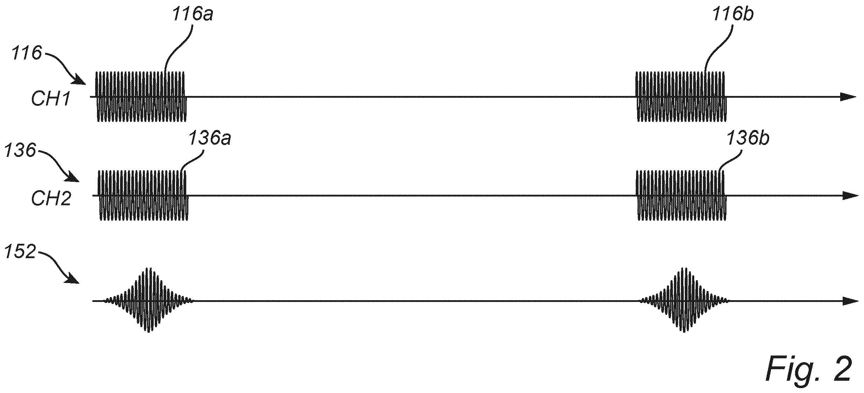

Referring now to Fig. 2, intermittent current waveforms according to an embodiment is illustrated. According to the invention, continuous first and second signals 16, 36 as illustrated above in relation to Fig. 1, are not used. Rather, a first intermittent current waveform 116 is generated and output to a first pair of electrodes arranged in a first location in relation to a nerve and a second intermittent current waveform 136 is generated and output to a second pair of electrodes arranged in a second location in relation to a nerve.

The first intermittent current waveform 116 comprises a sequence of first pulses, here illustrated as two pulses 116a, 116b. It should be realized that the sequence of first pulses may comprise a much larger number of pulses, such as more than 10 pulses or more than 100 pulses. The first intermittent current waveform 116 may be a zero-level signal or low amplitude signal between the pulses 116a, 116b and the waveform 116 is thus called "intermittent" since the waveform is a merely intermittently providing a non-zero signal level or a sufficient level for causing a stimulation of the nerve.

The first intermittent current waveform 116 may comprise a first frequency within the first pulses 116a, 116b, as illustrated by the alternating signal within the first pulses 116a, 116b.

The second intermittent current waveform 136 comprises a sequence of second pulses, here illustrated as two pulses 136a, 136b. It should be realized that the sequence of second pulses may comprise a much larger number of pulses, such as more than 10 pulses or more than 100 pulses. The number of pulses of the sequence of first pulses may be equal to the number of pulses of the sequence of second pulses. The second intermittent current waveform 136 may be a zero-level signal or low amplitude signal between the pulses 136a, 136b and the waveform 136 is thus called "intermittent" since the waveform is a merely intermittently providing a non-zero signal level or a sufficient level for causing a stimulation of the nerve.

The second intermittent current waveform 136 may comprise a second frequency within the second pulses 136a, 136b, as illustrated by the alternating signal within the second pulses 136a, 136b.

The first intermittent current waveform 116 and the second intermittent current waveform 136 being output by the first pair of electrodes and the second pair of electrodes may cause an interferential signal 152 to be formed. As illustrated in Fig. 2, the interferential signal 152 may comprise a pulse wherein the amplitude of the interferential signal 152 is modulated to define the beat frequency being a difference between the frequency within the first pulses 116a, 116b and the frequency within the second pulses 136a, 136b.

The duration of the first pulses 116a, 116b and the second pulses 136a, 136b may be so short as to correspond to a single or a few periods of the beat frequency. The generation and output of the first intermittent current waveform 116 and the generation and output of the second intermittent current waveform 136 may be synchronized so that the first pulses 116a, 116b are output simultaneously with the second pulses 136a, 136b in order to form the desired interferential signal 152. Further, a phase of the signal of the first frequency may be synchronized or controlled in relation to phase of the signal of the second frequency in order to control a desired phase of the interferential signal 152.

Thanks to the use of the first and second intermittent current waveforms 116, 136, stimulation of a nerve may be provided in an energy-efficient manner with no power being output in the first and second intermittent current waveforms 116, 136 between pulses when stimulation is not desired.

The interferential signal 152 has a low frequency compared to the first frequency and the second frequency. Since the nerve is more sensitive to stimulation by lower frequencies, this implies that the interferential signal 152 may stimulate the nerve, whereas the individual first and second intermittent current waveforms 116, 136 having a much higher frequency than the interferential signal 152 do not affect the nerve. Hence, even though the signals of the first intermittent current waveform 116 and the second intermittent current waveform 136 is much stronger close to the surface of the nerve (close to the electrodes), stimulation of the nerve may still be localized to locations deep within the nerve where interference between the first and the second intermittent current waveforms 116, 136 takes place.

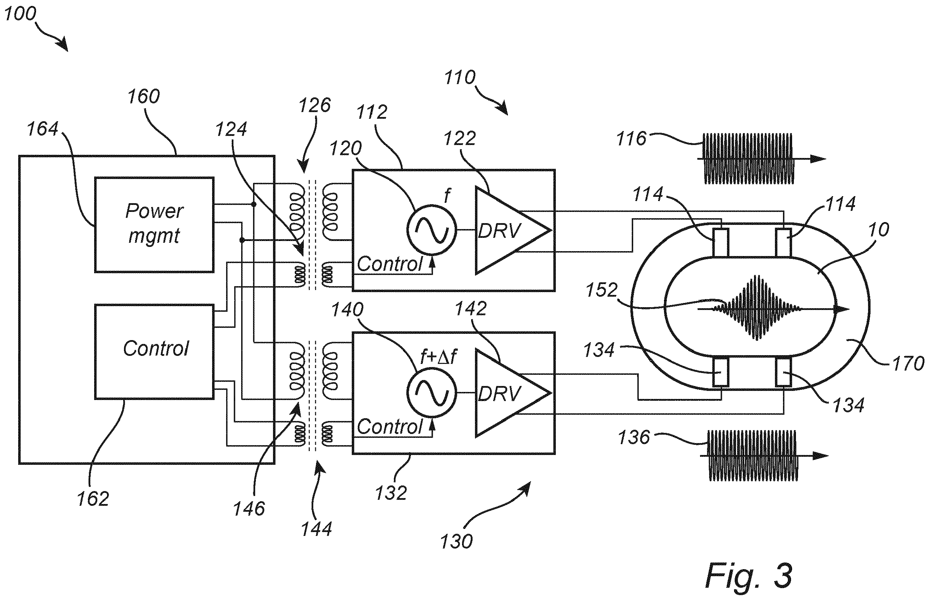

Referring now to Fig. 3, a device 100 according to a first embodiment for stimulating a nerve 10 will be described.

The device 100 comprises a first stimulation generating unit 110 and a second stimulation generating unit 130. The first stimulation generating unit 110 and the second stimulation generating unit 130 are separate from each other such that the generation and output of stimulation signals from the stimulation generating units 110, 130 may be individually provided without crosstalk between the stimulation generating units 110, 130.

The first stimulation generating unit 110 comprises a first current generator 112 and a first pair of electrodes 114 connected to the first current generator 112. The first current generator 112 is configured to generate the first intermittent current waveform 116 and to output the first intermittent current waveform 116 to the first pair of electrodes 114.

The second stimulation generating unit 130 comprises a second current generator 132 and a second pair of electrodes 134 connected to the second current generator 132. The second current generator 132 is configured to generate the second intermittent current waveform 136 and to output the second intermittent current waveform 136 to the second pair of electrodes 134.

As discussed above in relation to Fig. 2, the first stimulation generating unit 110 and the second stimulation generating unit 130 are configured to generate and output the first intermittent current waveform 116 and the second intermittent current waveform 136 with a difference in frequency of a first pulse 116a in the sequence of first pulses 116a, 116b and a second pulse 136a in the sequence of second pulses 136a, 136b such that the device 100 is configured to stimulate the nerve 10 using interferential stimulation based on interference between the first intermittent current waveform 116 and the second intermittent current waveform 136.

The device 100 further comprises a control unit 160. The control unit 160 may be configured to provide control signals to the first stimulation generating unit 110 and the second stimulation generating unit 130 for controlling generation of the first intermittent current waveform 116 and generation of the second intermittent current waveform 136, respectively. The control unit 160 may further be configured to synchronize the first stimulation generating unit 110 and the second stimulation generating unit 130 such that the first pulses 116a, 116b are output simultaneously or almost simultaneously with the second pulses 136a, 136b with a desired time relation to each other. The control unit 160 may thus ensure that a desired interferential signal 152 is generated for providing desired interferential stimulation of the nerve 10.

The first stimulation generating unit 110 and the second stimulation generating unit 130 may be configured to receive the same clock signal. The first stimulation generating unit 110 and the second stimulation generating unit 130 may further be configured to receive a control signal from the control unit 160 so as to control output of the first intermittent current waveform 116 and the second intermittent current waveform 136 in relation to the same clock signal. In this manner, the first stimulation generating unit 110 and the second stimulation generating unit 130 are synchronized.

The first pair of electrodes 114 and the second pair of electrodes 134 may be configured to be arranged in relation to a cross-section of the nerve 10 perpendicular to a longitudinal direction of the nerve 10. The first pair of electrodes 114 may be configured to be arranged at an opposite side of a circumference of the nerve 10 to the second pair of electrodes 134. However, individual electrodes within a pair of electrodes 114, 134 may still be configured to be arranged with a distance in-between such that the current waveform received by the electrodes will propagate through nerve tissue between the electrodes.

Thus, the first pair of electrodes 114 may be associated with a first part of the circumference of the nerve 10 and the second pair of electrodes 134 may be associated with a second part of the circumference of the nerve 10. This implies that the portions of the cross-section of the nerve in which the first intermittent current waveform 116 and the second intermittent current waveform 136, respectively, define electric fields are quite different so as to allow interferential stimulation deep within the nerve 10.

However, according to an alternative, the electrodes of the first pair of electrodes 114 may be configured to be alternatingly arranged with the electrodes of the second pair of electrodes 134 around the circumference of the nerve 10. Different arrangements of the first pair of electrodes 114 and the second pair of electrodes 134 in relation to the nerve 10 may be used depending on a location within the nerve 10 at which stimulation is desired.

According to another alternative, the electrodes of the first pair of electrodes 114 may be displaced in relation to each other along a longitudinal direction of the nerve 10. The electrodes of the second pair of electrodes 134 may also be displaced in relation to each other along a longitudinal direction of the nerve 10. The first pair of electrodes 114 may be displaced from the second pair of electrodes 134 along the circumference of the nerve 10, but the electrodes of the first and second pair of electrodes 114, 134 may be arranged at common positions in relation to the longitudinal direction of the nerve 10. This implies that an interferential signal 152 may be provided along the longitudinal direction of the nerve 10, which may be useful for stimulating the nerve 10. However, it may be more difficult to select a desired location within the cross-section of the nerve 10 when the electrodes of the pairs of electrodes 114, 134 are displaced in the longitudinal direction of the nerve 10.

As illustrated in Fig. 3, the device 100 may comprise a cuff 170 in which the first pair of electrodes 114 and the second pair of electrodes 134 are mounted. The cuff 170 may facilitate arrangement of the first and second pair of electrodes 114, 134 in contact with a surface of the nerve 10.

The first pair of electrodes 114 and the second pair of electrodes 134 may be mounted in the cuff 170 with a fixed relation between the electrodes. This implies that the cuff 170 may provide a well-defined relation between the electrodes providing a simple manner of arranging all the electrodes with a desired relationship to each other and to the nerve 10.

As mentioned above, the control unit 160 may be configured to provide control signals to the first current generator 112 and to the second current generator 132, respectively, for controlling the generation of the first intermittent current waveform 116 and the generation of the second intermittent current waveform 136. The control signals to the first current generator 112 and to the second current generator 132 may modulate the first intermittent current waveform 116 and the second intermittent current waveform 136, respectively, for controlling the interferential stimulation provided by the device 100.

The control unit 160 may be configured to provide control signals for modulating a frequency of the first pulses 116a, 116b and a frequency of the second pulses 136a, 136b. This implies that the beat frequency may be changed.

The control unit 160 may be configured to provide control signals for modulating an amplitude of the first pulses 116a, 116b and an amplitude of the second pulses 136a, 136b. This implies that a location within the nerve 10 having a maximum amplitude of the interferential signal 152 may be moved. The location having maximum amplitude of the interferential signal 152 is located closer to the pair of electrodes outputting a weaker signal than to the pair of electrodes outputting a stronger signal.

The control unit 160 may be configured to provide control signals for modulating a time instant for start of the first pulses 116a, 116b and a time instant for start of the second pulses 136a, 136b. Thus, a phase relationship between the first pulses 116a, 116b and the second pulses 136a, 136b may be controlled for controlling a waveform of the interferential signal 152.

The control unit 160 may comprise a stimulation controller 162 which is configured to generate and transmit the control signals to the first stimulation generating unit 110 and the second stimulation generating unit 130.

According to an embodiment, the stimulation controller 162 may comprise a processing unit for executing an application program for controlling the generation and timing of control signals. The stimulation controller 162 may further comprise a memory storing the application program and storing settings for controlling the generation and timing of control signals. The stimulation controller 162 may be configured to receive new settings for altering the control by the stimulation controller 162. The stimulation controller 162 may also or alternatively comprise several versions of settings to allow selection of different settings depending on a desired stimulation. The stimulation controller 162 may further comprise a clock or may be configured to receive a clock signal as input for timing of control signals.

According to an alternative, the stimulation controller 162 may comprise an application-specific integrated circuit (ASIC) for controlling the generation and timing of control signals.

The control unit 160 may further comprise a power management unit 164, which may be configured to control use of power by the device 100. The power management unit 164 may be configured to send control signals to other components of the device 100 for controlling states of components of the device 100. The power management unit 164 may be configured to control whether a component is turned on or off, such as controlling whether a component is in an awake state, a sleep state, or in a completely turned off state. A component in sleep state may consume some power, whereas a component which is completely turned off does not consume power.

The first stimulation generating unit 110 and the second stimulation generating unit 130 may be configured to generate a frequency of the first pulses 116a, 116b and a frequency of the second pulses 136a, 136b, respectively, being in a range of 500 Hz - 1 MHz, such as 50 kHz - 1 MHz.

The first stimulation generating unit 110 and the second stimulation generating unit 130 may further be configured to generate the first intermittent current waveform 116 and the second intermittent current waveform 136 with a difference in frequency in a range of 1 Hz - 10 kHz, such as 1 - 5 kHz. Thus, the beat frequency of the interferential signal 152 may be in the range of 1 Hz - 10 kHz, such as 1 - 5 kHz.

The first frequency and the second frequency generated by the first stimulation generating unit 110 and the second stimulation generating unit 130 may thus be sufficiently high so as not to affect the nerve 10, such as being higher than 500 Hz or higher than 50 kHz. The difference between the first frequency and the second frequency, i.e., the beat frequency, may be set so as to be adapted to provide proper stimulation of the nerve 10.

The signal of the first intermittent current waveform 116 in the first pulses 116a, 116b and the signal of the second intermittent current waveform 136 in the second pulses 136a, 136b may be sinusoidal signals. This implies that no net charges are delivered to the living being by the first intermittent current waveform 116 and the second intermittent current waveform 136, respectively. Hence, accumulation of net charges in the living being is avoided, which may otherwise negatively affect the living being.

The stimulation controller 162 may be configured to control the first stimulation generating unit 110 and the second stimulation generating unit 130 to control the device 100 to provide stimulation of the nerve 10 according to a stimulation paradigm. The stimulation controller 162 may thus store settings of the stimulation paradigm and may be configured to control the first stimulation generating unit 110 and the second stimulation generating unit 130 to generate and output the first intermittent current waveform 116 and the second intermittent current waveform 136 for providing the desired stimulation paradigm to the nerve 10.

Depending on the purpose of stimulating the nerve 10, different settings of the stimulation paradigm may be used. Typically, the stimulation paradigm may involve providing pulses for stimulating the nerve 10 having a pulse width in a range of 100 - 5000 µs, such as 100 - 500 µs. The pulse width relates to a pulse of the interferential signal 152, which is achieved by providing corresponding pulse widths of the first pulses 116a, 116b and the second pulses 136a, 136b. The stimulation paradigm may further involve repeating the pulses with a frequency of 1 - 100 Hz, such as 20 - 30 Hz. This is a repetition frequency of repeating the pulses and should not be confused with the beat frequency of the interferential signal 152.

The stimulation paradigm may further involve using an "on time" and "off time". Thus, during a session of the stimulation paradigm, stimulation of the nerve 10 is provided during a period of time ("on time") using the settings of the pulse width and repetition frequency of pulses. Thereafter, no stimulation is provided during a period of time ("off time"), followed by again stimulating the nerve 10 during another period of time ("on time"). The number of "on time" periods during a session of stimulation of the nerve 10 may be in a range of 10 - 1000. The stimulation paradigm may use "off time" periods in order to avoid habituation of the stimulation, diminishing an effect of stimulation of the nerve 10. Stimulation may be provided for instance on a daily basis such that one or more sessions of stimulation of the nerve 10 may be provided each day. The stimulation of the nerve 10 may be provided during a very long overall period, such that the device 100 may be used for months or years. It should also be realized that the stimulation may be ongoing in a very long session with alternating "on time" periods and "off time" periods. However, stimulation is only provided during a small percentage of time, such as in a duty cycle of less than 10% daily.

In some embodiments, a duration of an "on time" period may be in a range of 0.5 s - 30 minutes. In some embodiments, a duration of an "off time" period may be in a range of 10 s - 1 hour. It should be realized that duration of "on time" period and "off time" periods may vary heavily with the purpose of stimulating the nerve 10.

A current amplitude of the pulses of the interferential signal 152 may be in a range of 0.1 - 10 mA. An amplitude of the first pulses 116a, 116b of the first interferential current waveform 116 and an amplitude of the second pulses 136a, 136b of the second interferential current waveform 136 may be smaller than the amplitude of the pulses of the interferential signal 152, such as approximately half of the amplitude of the pulses of the interferential signal 152.

According to an embodiment, the device 100 may be configured to provide stimulation of the nerve 10 according to a stimulation paradigm for treating epilepsy. In an example, the device 100 may be configured to stimulate the nerve 10 using pulses having a pulse width of 500 µs, a repetition frequency of 30 Hz, a duration of "on time" period of 30 s, and a duration of "off time" period of 5 minutes.

According to another embodiment, the device 100 may be configured to provide stimulation of the nerve 10 according to a stimulation paradigm for treating depression. In an example, the device 100 may be configured to stimulate the nerve 10 using pulses having a pulse width of 500 µs, a repetition frequency of 20 Hz, a duration of "on time" period of 30 s, and a duration of "off time" period of 5 minutes.

The power management unit 164 may be configured to control the first stimulation generating unit 110 and the second stimulation generating unit 130 for turning on and off the first stimulation generating unit 110 and the second stimulation generating unit 130. Thus, the power management unit 164 may control the first stimulation generating unit 110 and the second stimulation generating unit 130 to be turned on when the first pulses 116a, 116b of the first intermittent current waveform 116 and when the second pulses 136a, 136b of the second intermittent current waveform 136, respectively, are to be generated. Hence, the first stimulation generating unit 110 and the second stimulation generating unit 130 may be turned off between generation of individual pulses in the sequence of first pulses 116a, 116b and in the sequence of second pulses 136a, 136b. This implies that signals for stimulating the nerve 10 may be generated in a very energy-efficient manner.

The power management unit 164 may be configured to control the first stimulation generating unit 110 and the second stimulation generating unit 130 to assume a sleep state between generation of individual pulses in the sequence of first pulses 116a, 116b and in the sequence of second pulses 136a, 136b, such that the first stimulation generating unit 110 and the second stimulation generating unit 130 are not completely turned off and may quickly assume an awake state.

The power management unit 164 may further be configured to control the first stimulation generating unit 110 and the second stimulation generating unit 130 to assume a completely turned off state during "off time" periods. This implies that the first stimulation generating unit 110 and the second stimulation generating unit 130 need not consume any power during the "off time" periods.

The first current generator 112 and the second current generator 132 will now be described in further detail. The first current generator 112 may comprise an oscillator 120 and an amplifier 122 and the second current generator 132 may comprise an oscillator 140 and an amplifier 142.

The first current generator 112 and the second current generator 132 may be identical and for clarity and brevity only the first current generator 112 will be described below, but it should be realized that the description below also applies to the second current generator 132. However, the first current generator 112 may be controlled to generate a signal having a first frequency and the second current generator 122 may be controlled to generate a signal having a second frequency different from the first frequency.

The oscillator 120 of the first current generator 112 may be configured to generate an alternating signal, such as a sinusoidal signal. The oscillator 120 may be any type of electronic oscillator for generating the alternating signal.

The oscillator 120 may receive a control signal from the control unit 160 for controlling the frequency of the signal output by the oscillator 120, the amplitude of the signal output by the oscillator 120 and/or a timing of output of the signal by the oscillator 120.

The oscillator 120 may be connected to the amplifier 122 such that the alternating signal output by the oscillator 120 is received by the amplifier 122. The amplifier 122 may be configured to amplify the signal output by the oscillator 120 and output a source current and a sink current.

The amplifier 122 may be connected to the first pair of electrodes 114 such that the amplifier may output the source current to a first electrode and the sink current to a second electrode in the first pair of electrodes 114 for forming the first intermittent current waveform 116.

The amplifier 122 may be configured to receive a control signal from the control unit 160 for controlling gain of the amplifier 122 so as to control the amplitude of the pulses 116a, 116b of the first intermittent current waveform 116. Thus, the amplitude may be controlled by controlling the amplitude of the signal output by the oscillator 120 and/or by controlling the gain of the amplifier 122.

The first stimulation generating unit 110 and the second stimulation generating unit 130 may be galvanically isolated from each other. This implies that any crosstalk between the first stimulation generating unit 110 and the second stimulation generating unit 130 may be avoided. Thus, the interferential signal 152 for causing stimulation of the nerve 10 may be defined by the first intermittent current waveform 116 and the second intermittent current waveform 136 being independently generated.

Also, the source current generated by the first current generator 112 and the source current generated by the second current generator 132 may have a floating potential in relation to each other. This further avoids crosstalk between the first stimulation generating unit 110 and the second stimulation generating unit 130.

As shown in Fig. 3, the first stimulation generating unit 110 and the second stimulation generating 130 may be galvanically isolated from each other by inductive couplings 124, 126, 144, 146 being used for transferring control signals from the control unit 160 to the first stimulation generating unit 110 and the second stimulation generating unit 130, respectively.

Thus, pairs of coils may be used for transferring control signals from the control unit 160 to the first stimulation generating unit 110 and the second stimulation generating unit 130, respectively.

A first pair of coils 124 may be used for transferring control signals from the stimulation controller 162 to the first stimulation generating unit 110 for controlling generation of the first intermittent current waveform 116. A second pair of coils 126 may be used for transferring control signals from the power management unit 164 to the first stimulation generating unit 110 for controlling a state of the first stimulation generating unit 110.

A third pair of coils 144 may be used for transferring control signals from the stimulation controller 162 to the second stimulation generating unit 130 for controlling generation of the second intermittent current waveform 136. A fourth pair of coils 146 may be used for transferring control signals from the power management unit 164 to the second stimulation generating unit 130 for controlling a state of the second stimulation generating unit 130.

The device 100 may be configured to be implanted in the living being, such as a human being. The first pair of electrodes 114 and the second pair of electrodes 134 are preferably configured to be in contact with the nerve 10 for providing accurate control of signals transmitted into the nerve 10 and avoiding attenuation of signals from the first pair of electrodes 114 and the second pair of electrodes 134 before reaching the nerve 10.

The first current generator 112 and the second current generator 132 are preferably also implanted in the living being. This implies that wires connecting the first current generator 112 to the first pair of electrodes 114 and wires connecting the second current generator 132 to the second pair of electrodes 134 may be short so as to provide good quality of signals provided to the first pair of electrodes 114 and the second pair of electrodes 134.

The control unit 160 is preferably also implanted in the living being. The control unit 160 may transfer control signals to the first stimulation generating unit 110 and the second stimulation generating unit 130 through wired communication (via the inductive couplings 124, 126, 144, 146). Thus, the control unit 160 may be implanted in the living being to avoid wires extending from an implant to a unit external to the body of the living being.

The control unit 160, the first current generator 112, and the second current generator 132 may be arranged within a common housing, which may be positioned close to the nerve 10 when the device 100 is implanted. The housing may provide an encasing of circuitry of the device 100 so as to protect the circuitry and to provide an appropriate outer surface for implantation in the living being.

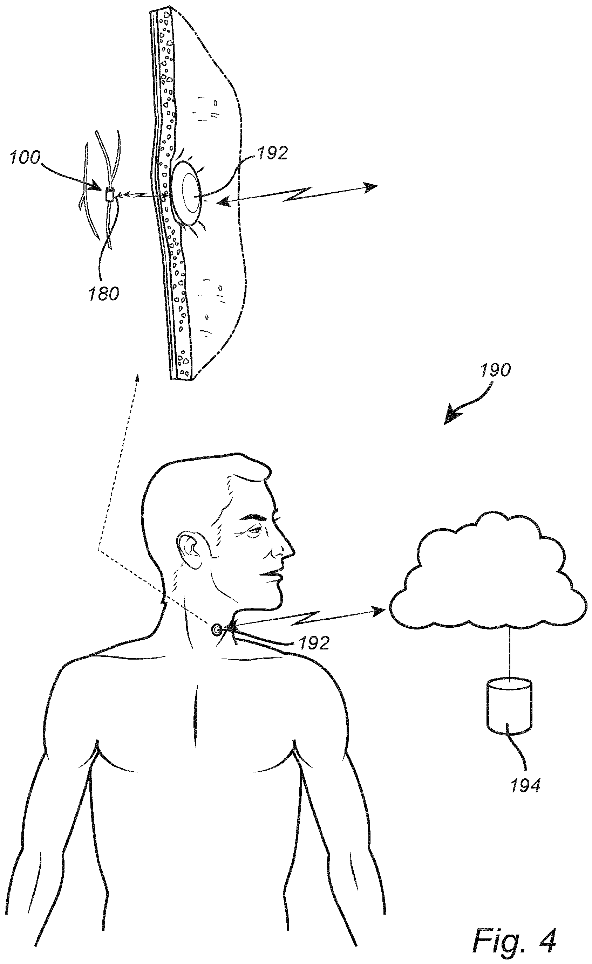

Referring now to Fig. 4, a system 190 will be the described. The system 180 comprises the implanted device 100. The implanted device 100 may further comprise a communication unit 180, which may be configured to communicate with an external device 192 of the system 190 through wireless communication.

The implanted device 100 may thus receive control signals from the external device 192 for changing parameters of the stimulation, such as parameters defining the stimulation paradigm, such as to control a location within the nerve 10 to be stimulated or a frequency, an amplitude, or a duration of a stimulation to be provided. The communication unit 180 may be provided within the control unit 160 or may be separately provided for providing wireless communication to the external device 192.

The external device 192 may be provided as a wearable for allowing use of short-range communication between the implanted device 100 and the external device 192. The external device 192 may be configured to be worn on the skin close to the implanted device 100. However, the external device 192 may alternatively be configured to be worn in any other suitable manner, such as the external device 192 being in form of a smartwatch provided with functionality for communicating with the implanted device 100. Alternatively, the external device 192 may be adapted to be arranged close to the living being, even if not being necessarily worn, such as the external device 192 being in form of a mobile phone provided with functionality for communicating with the implanted device 100.

The external device 192 may function as an intermediate device for allowing the implanted device 100 to receive and transmit information to a remote device 194. Thus, the external device 192 may enable communication between the implanted device 100 and a remote device 194 arranged anywhere, e.g., by means of communication between the external device 192 and the remote device 194 through a telecommunication or computer network, such as the Internet.

The external device 192 and/or the remote device 194 may provide information to the implanted device 100 for configuring the implanted device 100 such as for controlling parameters of the stimulation.

The implanted device 100 may further be configured to transmit information to the external device 192 and/or the remote device 194 regarding stimulations performed, which may be used as input for controlling future stimulations.

The external device 192 and the implanted device 100 may further be configured to provide wireless charging of the implanted device 100. Thus, the implanted device 100 may be wirelessly charged so as to increase lifetime of the implanted device 100.

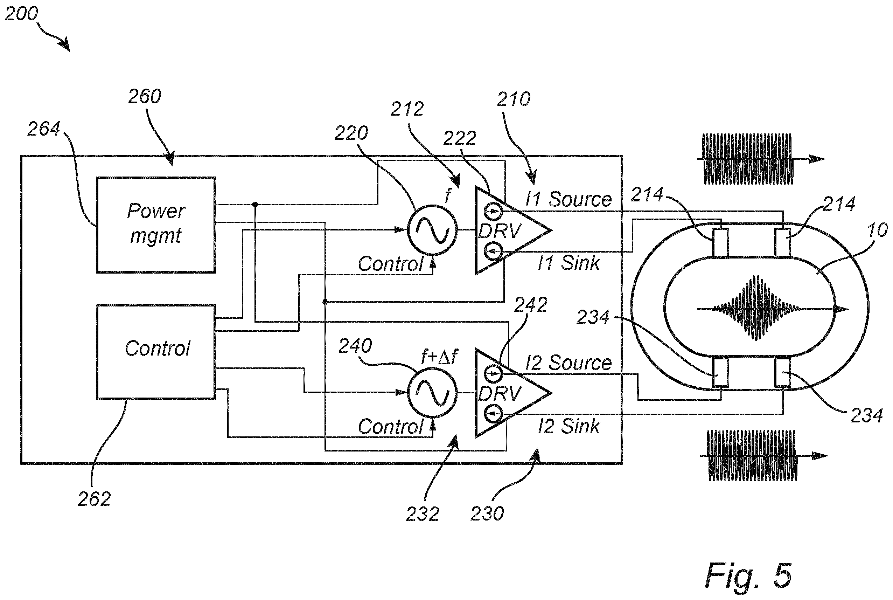

Referring now to Fig. 5, a device 200 according to a second embodiment for stimulating a nerve 10 will be described. Similar to the device 100 according to the first embodiment, the device 200 according to the second embodiment comprises a first stimulation generating unit 210 and a second stimulation generating unit 230, each comprising a current generator 212, 232 and a pair of electrodes 214, 234. The current generators 212, 232 each comprise an oscillator 220, 240 and an amplifier 222, 242. The device further comprises a control unit 260 comprising a stimulation controller 262 and a power management unit 264.

Below only the differences between the device 200 according to the second embodiment and the device 100 according to the first embodiment will be discussed. Apart from the differences discussed below, the device 200 according to the second embodiment may function in the same way as described above for the device 100 according to the first embodiment. It should also be realized that the device 200 may be used instead of the device 100 in a system 180 as described above.

In the device 200, the first stimulation generating unit 210 and the second stimulation generating unit 230 are not galvanically isolated. The device 200 is still configured to avoid crosstalk between the first stimulation generating unit 110 and the second stimulation generating unit 130.

The stimulation controller 262 is connected to the oscillator 222 of the first current generator 212 for providing a control signal which may set parameters of the oscillator 222 (frequency, amplitude). The stimulation controller 262 is also separately connected to the oscillator 222 for triggering generation of a first pulse of the first intermittent current waveform 216.

The stimulation controller 262 is further connected to the oscillator 242 of the second current generator 232 for providing a control signal which may set parameters of the oscillator 242 (frequency, amplitude). The stimulation controller 262 is also separately connected to the oscillator 242 for triggering generation of a second pulse of the second intermittent current waveform 236.

The stimulation controller 262 is separately connected to the oscillator 222 of the first current generator 212 and to the oscillator 242 of the second current generator 232.

According to an alternative, the stimulation controller 262 is connected to the oscillators 222, 242 for setting the parameters. The stimulation controller 262 is further configured to provide a digital clock source to the first current generator 212 and the second current generator 222. The digital clock source may control the first and the second intermittent current waveforms, respectively, to be generated based on a memory storing the waveform, which is converted to an analog signal.

Further, the power management unit 264 is connected to the first stimulation generating unit 210 and the second stimulation generating unit 230 for controlling start of a source current signal and start of a sink current signal provided by the first stimulation generating unit 210 and the second stimulation generating unit 230, respectively.

This implies that the first stimulation generating unit 210 may be controlled by two control signals for setting the frequency and controlling the sink current signal, respectively, and the second stimulation generating unit 230 may be controlled by two control signals for setting the frequency and controlling the sink current signal, respectively. This may be used for avoiding crosstalk between the first stimulation generating unit 210 and the second stimulation generating unit 230.

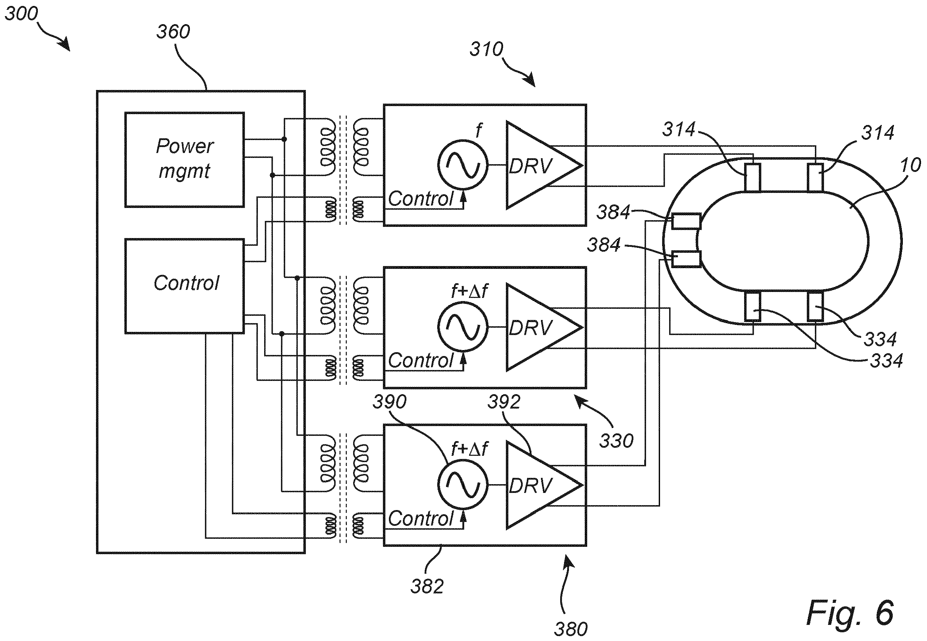

Referring now to Fig. 6, a device 300 according to a third embodiment for stimulating a nerve 10 will be described. The device 300 is identical to the device 100 according to the first embodiment, except that a third stimulation generating unit 380 is added in addition to the first stimulation generating unit 310 and the second stimulation generating unit 330. The third stimulation generating unit 380 is also connected to the control unit 360 for controlling generation of a third intermittent waveform.

Although the device 300 is illustrated here based on connection between the control unit 360 and the stimulation generating units 310, 330, 380 in a similar manner as discussed above for the device 100 according to the first embodiment, it should be realized that the connection between the control unit 360 and the stimulation generating units 310, 330, 380 may instead be provided in a similar manner as discussed above for the device 200 according to the second embodiment.

Below only the differences between the device 200 according to the second embodiment and the device 100 according to the first embodiment will be discussed. Apart from the differences discussed below, the device 200 according to the second embodiment may function in the same way as described above for the device 100 according to the first embodiment. It should also be realized that the device 300 may be used instead of the device 100 in a system 180 as described above.

The third stimulation generating unit 380 may be configured in a common manner as the first stimulation generating unit 310 and the second stimulation generating unit 380. Thus, the third stimulation generating unit 380 may comprise a third current generator 382, having an oscillator 390 and an amplifier 392 for generating a third intermittent current waveform comprising a sequence of third pulses.

The third stimulation generating unit 380 may further comprise a third pair of electrodes 384 connected to the third current generator 382 for receiving the third intermittent current waveform.

The control unit 360 may be connected to the third stimulation generating unit 380 for providing independent control of each of the first stimulation generating unit 310, the second stimulation generating unit 330 and the third stimulation generating unit 380.

The third stimulation generating unit may be configured to provide a different frequency of the signal of the third pulses from the frequencies of the first pulses and the second pulses, respectively. Alternatively, the frequency of the signal of the third pulses may be the same as the frequency of the first pulses or the frequency of the second pulses.

The third pair of electrodes 384 may be arranged at a different location in relation to the nerve 10 compared to the first pair of electrodes 314 and the second pair of electrodes 334. All of the electrodes of the first pair of electrodes 314, the second pair of electrodes 334, and the third pair of electrodes 384 may be arranged in relation to a common cross-section of the nerve 10 with different placements around the circumference of the nerve 10. The first pair of electrodes 314 may be associated with a first part of the circumference of the nerve 10, the second pair of electrodes 334 may be associated with a second part of the circumference of the nerve 10, and the third pair of electrodes 384 may be associated with a third part of the circumference of the nerve 10. Alternatively, the electrodes of different pairs of electrodes may be alternatingly arranged around the circumference of the nerve 10.

According to an alternative, the individual electrodes of the pairs of electrodes 314, 334, 384 may be displaced in relation to each other along a longitudinal direction of the nerve 10. The first pair of electrodes 314, the second pair of electrodes 334, and the third pair of electrodes 384 may be displaced in relation to each other along the circumference of the nerve 10, but the electrodes of the first, second, and third pairs of electrodes 314, 334, 384 may be arranged at common positions in relation to the longitudinal direction of the nerve 10. This implies that an interferential signal may be provided along the longitudinal direction of the nerve 10.

The third stimulation generating unit 380 may be used such that the device 300 may control interferential stimulation based on the first intermittent current waveform, the second intermittent current waveform, and the third intermittent current waveform being simultaneously output. The interferential stimulation may thus be even more accurately controlled when the third intermittent current waveform may be used in addition to the first and second intermittent current waveforms for defining the interferential stimulation.

However, the device 300 may additionally or alternatively control interferential stimulation by the control unit 360 selectively activating stimulation generating units so as to select which two of the first intermittent current waveform, the second intermittent current waveform, and the third intermittent current waveform are to be used for defining interferential stimulation. In other words, the control unit 350 may be configured to select which electrodes that are to be used for outputting signals for defining the interferential stimulation.

Hence, the control unit 360 may be configured to modulate interferential stimulation by selecting which electrodes to be used for output of waveforms for stimulation of the nerve. The selection of which electrodes to be used may be used for controlling a location within the nerve 10 to be stimulated.

It should be realized that additional stimulation generating units may also be provided. This implies that further possibilities of controlling the interferential stimulation are provided for improving control of the interferential stimulation.

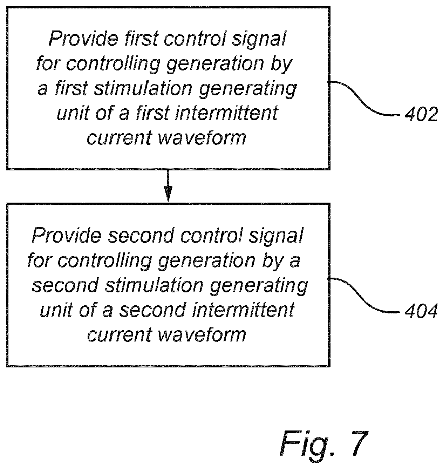

Referring now to Fig. 7, a method for controlling stimulation of a nerve 10 will be described.

The method comprises providing 402 a first control signal for controlling a first stimulation generating unit 110, 210, 310. The control signal controls generation by the first stimulation generating unit 110, 210, 310 of a first intermittent current waveform comprising a sequence of first pulses.

The method further comprises providing 404 a second control signal for controlling a second stimulation generating unit 130, 230, 330. The control signal controls generation by the second stimulation generating unit 130, 230, 330 of a second intermittent current waveform comprising a sequence of second pulses.

The control signals to the first stimulation generating unit 110, 210, 310 and the second stimulation generating unit 130, 230, 330 controls generation of the first intermittent current waveform and the second intermittent current waveform with a difference in frequency of a first pulse in the sequence of first pulses and a second pulse in the sequence of second pulses.

The control signals are synchronized for synchronizing the first stimulation generating unit 110, 210, 310 and the second stimulation generating unit 130, 230, 330. This implies that the first intermittent current waveform and the second intermittent current waveform may be generated with a desired synchronization such that a desired interferential stimulation may be generated based on the first intermittent current waveform and the second intermittent current waveform.

The method may further comprise controlling the first stimulation generating unit 110, 210, 310 and the second stimulation generating unit 130, 230, 330 for turning on and off the first stimulation generating unit 110, 210, 310 and the second stimulation generating unit 130, 230, 330.

The control of the first stimulation generating unit 110, 210, 310 and the second stimulation generating unit 130, 230, 330 may ensure that the first stimulation generating unit 110, 210, 310 and the second stimulation generating unit 130, 230, 330 are turned off between generation of individual pulses in the sequence of first pulses and in the sequence of second pulses.

The method may further comprise modulating the first intermittent current waveform and the second intermittent current waveform by providing control signals to the first stimulation generating unit 110, 210, 310 and the second stimulation generating unit 130, 230, 330, respectively. The control signals may modulate the first intermittent current waveform and the second intermittent current waveform by modulating a frequency of the first pulses and a frequency of the second pulses, respectively, by modulating an amplitude of the first pulses and an amplitude of the second pulses, respectively, and/or by modulating a time instant for start of the first pulses and a time instant for start of the second pulses, respectively.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A device (100; 200; 300) for stimulating a nerve (10) of a living being, said device (100; 200; 300) comprising:
a first stimulation generating unit (110; 210; 310) comprising a first current generator (112; 212) and a first pair of electrodes (114; 214; 314) configured to be arranged in a first location in relation to the nerve (10), wherein the first current generator (112; 212) is configured to generate and output a first intermittent current waveform (116) comprising a sequence of first pulses (116a, 116b) to the first pair of electrodes (114; 214; 314);
a second stimulation generating unit (130; 230; 330) comprising a second current generator (132; 232) and a second pair of electrodes (134; 234; 334) configured to be arranged in a second location in relation to the nerve (10), wherein the second current generator (132; 232) is configured to generate and output a second intermittent current waveform (136) comprising a sequence of second pulses (136a, 136b) to the second pair of electrodes (134; 234; 334);
wherein the first stimulation generating unit (110; 210; 310) and the second stimulation generating unit (130; 230; 330) are configured to generate and output the first intermittent current waveform (116) and the second intermittent current waveform (136) with a difference in frequency of a first pulse in the sequence of first pulses (116a, 116b) and a second pulse in the sequence of second pulses (136a, 136b) such that the device (100; 200; 300) is configured to stimulate the nerve (10) using interferential stimulation based on interference between the first intermittent current waveform (116) and the second intermittent current waveform (136); and
a control unit (160; 260; 360) configured to control the first intermittent current waveform (116) and the second intermittent current waveform (136) for controlling the interferential stimulation and configured to synchronize the first stimulation generating unit (110; 210; 310) and the second stimulation generating unit (130; 230; 330) for controlling output of the first pulses and the second pulses.

2. The device according to claim 1, wherein the device (100; 200; 300) is configured to be implanted in the living being with the first pair of electrodes (114; 214; 314) and the second pair of electrodes (134; 234; 334) arranged at different locations in relation to the nerve (10).

3. The device according to claim 2, wherein the device (100; 200; 300) comprises a cuff (170) configured to be arranged surrounding the circumference of the nerve (10), wherein the first pair of electrodes (114; 214; 314) and the second pair of electrodes (134; 234; 334) are mounted in or on the cuff (170).

4. The device according to any one of the preceding claims, wherein the control unit (160; 260; 360) is configured to control the first stimulation generating unit (110; 210; 310) and the second stimulation generating unit (130; 230; 330) for turning on and off the first stimulation generating unit (110; 210; 310) and the second stimulation generating unit (130; 230; 330), wherein the control unit (160; 260; 360) is configured to turn off the first stimulation generating unit (110; 210; 310) and the second stimulation generating unit (130; 230; 330) between generation of individual pulses in the sequence of first pulses (116a, 116b) and in the sequence of second pulses (136a, 136b).

5. The device according to any one of the preceding claims, wherein the control unit (160; 260; 360) is configured to modulate the first intermittent current waveform (116) and the second intermittent current waveform (136) by modulating a frequency of the first pulses and a frequency of the second pulses, respectively, by modulating an amplitude of the first pulses and an amplitude of the second pulses, respectively, and/or by modulating a time instant for start of the first pulses and a time instant for start of the second pulses, respectively.

6. The device according to any one of the preceding claims, wherein the first stimulation generating unit (110; 210; 310) and the second stimulation generating unit (130; 230; 330) are configured to generate a frequency of the first pulse and a frequency of the second pulse, respectively, being in a range of 500 Hz - 1 MHz, such as 50 kHz - 1 MHz.

7. The device according to any one of the preceding claims, wherein the first stimulation generating unit (110; 210; 310) and the second stimulation generating unit (130; 230; 330) are configured to generate the first intermittent current waveform (116) and the second intermittent current waveform (136) with a difference in frequency in a range of 1 Hz - 10 kHz, such as 1 - 5 kHz.

8. The device according to any one of the preceding claims, wherein each of the first current generator (112; 212) and the second current generator (132; 232) comprises an oscillator (120, 140; 220, 240), which is configured to generate an alternating signal and an amplifier (122, 142; 222, 242), which is configured to receive the alternating signal from the oscillator (120, 140; 220, 240) and generate a source current to a first electrode and a sink current to a second electrode in the first pair of electrodes (114, 214; 314) and the second pair of electrodes (134; 234; 334), respectively.

9. The device according to any one of the preceding claims, wherein the control unit (160; 360) is configured to transfer control signals to the first stimulation generating unit (110; 310) and the second stimulation generating unit (130; 330) via inductive couplings (124, 126, 144, 146).

10. The device according to any one of claims 1-8, wherein the first stimulation generating unit (210) is configured to provide a source current signal to a first electrode in the first pair of electrodes (214) and to provide a sink current signal to a second electrode in the first pair of electrodes (214), wherein the control unit (260) is configured to transmit a first control signal to the first stimulation generating unit (210) for setting frequency of the first stimulation generating unit (210) and to transmit a second control signal to the first stimulation generating unit (210) for controlling start of the source current signal and the sink current signal.

11. The device according to any one of the preceding claims, wherein the first pulses and second pulses are sinusoidal signals.

12. The device according to any one of the preceding claims, further comprising a third stimulation generating unit (380) comprising a third current generator (382) and a third pair of electrodes (384) configured to be arranged in a third location in relation to the nerve (10), wherein the third current generator (382) is configured to generate and output a third intermittent current waveform comprising a sequence of third pulses to the third pair of electrodes (384), wherein the control unit (360) is further configured to control the third intermittent current waveform for controlling the interferential stimulation based on the first, second, and third intermittent current waveforms.

13. A system (190) for stimulating a nerve (10) of a living being, said system (180) comprising:
a device (100; 200; 300) according to any one of the preceding claims, wherein the device (100; 200; 300) is configured to be implanted in the living being and the device (100; 200; 300) comprises a communication unit (180) for wireless communication;
an external device (192) configured to communicate through wireless communication with the communication unit (180) of the device (100; 200; 300).

14. A method for controlling stimulation of a nerve of a living being, said method comprising:
providing (402) a first control signal for controlling a first stimulation generating unit comprising a first current generator and a first pair of electrodes arranged in a first location in relation to the nerve, wherein the control signal controls generation of a first intermittent current waveform comprising a sequence of first pulses;
providing (404) a second control signal for controlling a second stimulation generating unit comprising a second current generator and a second pair of electrodes arranged in a second location in relation to the nerve, wherein the control signal controls generation of a second intermittent current waveform comprising a sequence of second pulses;
wherein the control signals to the first stimulation generating unit and the second stimulation generating unit controls generation of the first intermittent current waveform and the second intermittent current waveform with a difference in frequency of a first pulse in the sequence of first pulses and a second pulse in the sequence of second pulses;
wherein the control signals are synchronized for synchronizing the first stimulation generating unit and the second stimulation generating unit.

15. The method according to claim 13, further comprising controlling the first stimulation generating unit and the second stimulation generating unit for turning on and off the first stimulation generating unit and the second stimulation generating unit, wherein the first stimulation generating unit and the second stimulation generating unit are turned off between generation of individual pulses in the sequence of first pulses and in the sequence of second pulses.
